# EUROPEAN PATENT APPLICATION

(11) **EP 3 708 108 A1**
(43) Date of publication of application: **16.09.2020**
(21) Application number: 18886484.7
(22) Date of filing: 12.09.2018
(51) Int. Cl.: A61B 90/30, A61B 90/20, G02B 21/06, G03B 15/00, G03B 15/02, G03B 15/03, G03B 17/56, H04N 5/222, H04N 5/225, H04N 5/235

(54) **MEDICAL CONTROL APPARATUS AND CONTROL METHOD**

(30) Priority: 06.12.2017 JP 2017234284
(71) Applicant: Sony Olympus Medical Solutions Inc., Hachioji-shi, Tokyo 192-0904 (JP)
(72) Inventor: OKABE, Yasuhiro, Hachioji-shi Tokyo 192-0904 (JP); SHIRAGA, Mitsuaki, Hachioji-shi Tokyo 192-0904 (JP); ISHIKAWA, Tomonori, Hachioji-shi Tokyo 192-0904 (JP)
(74) Representative: D Young & Co LLP
(86) International application number: PCT/JP2018/033814
(87) International publication number: WO 2019/111482

(57) **Abstract**

There is provided a medical control apparatus including an illumination control unit that performs, based on a detection result of brightness relating to imaging, a first control of controlling brightness of a light source of an observation device for observing an observation target and a second control of controlling brightness of an illumination apparatus that irradiates the observation target with illumination light from an outside of the observation device.

## Description

### Technical Field

The present disclosure relates to a medical control apparatus and a control method.

### Background Art

In recent years, in a medical site, a medical observation apparatus capable of enlarging and observing an observation target such as an affected part is used in some cases in order to support microsurgery (microsurgery) such as neurosurgery. Examples of the medical observation apparatus include a medical observation apparatus including an optical microscope, and a medical observation apparatus including an imaging device that functions as an electronic imaging microscope. Hereinafter, the medical observation apparatus including the optical microscope will be referred to as an "optical medical observation apparatus". Hereinafter, the medical observation apparatus including the imaging device is referred to as an "electronic imaging medical observation apparatus" or simply as a "medical observation apparatus".

Under such circumstances, a technique relating to a surgical microscope (an example of an optical medical observation apparatus) having an illumination optical system has been developed. Examples of the above technique include a technique described in Patent Literature 1 described below.

### Citation List

### Patent Literature

Patent Literature 1: JP-A-H4-144554

### Disclosure of Invention

### Technical Problem

The electronic imaging medical observation apparatus obtains image quality equal to or higher than that of an optical medical observation apparatus, with the improvement of the image quality of the imaging device, the improvement of the image quality of the display device that displays a captured image, and the like. In addition, since a user using the electronic imaging medical observation apparatus (for example, a medical worker such as a surgeon or an assistant of the surgeon) does not need to look into an eyepiece constituting the optical microscope as in case of using the optical medical observation apparatus, it is possible to freely move the position of the imaging device. Therefore, by using the electronic imaging medical observation apparatus, there is an advantage that the surgery can be more flexibly supported by moving the position of the imaging device, and the use of electronic imaging medical observation apparatus at medical sites is increasing.

At a surgery site, there are various illuminations, for example, a light source of the imaging device included in the medical observation apparatus and an illumination apparatus such as a surgical light or a room light. In addition, at the surgery site, various light sources are manually controlled by a medical worker, which sometimes impairs the convenience of the medical worker.

The present disclosure proposes a novel and improved medical control apparatus and a control method which are capable of improving the convenience of a medical worker.

### Solution to Problem

According to the present disclosure, there is provided a medical control apparatus including an illumination control unit that performs, based on a detection result of brightness relating to imaging, a first control of controlling brightness of a light source of an observation device for observing an observation target and a second control of controlling brightness of an illumination apparatus that irradiates the observation target with illumination light from an outside of the observation device.

Moreover, according to the present disclosure, there is provided a control method executed by a medical control apparatus, the control method including a step of performing, based on a detection result of brightness relating to imaging, a first control of controlling brightness of a light source of an observation device for observing an observation target and a second control of controlling brightness of an illumination apparatus that irradiates the observation target with illumination light from an outside of the observation device.

### Advantageous Effects of Invention

According to the present disclosure, it is possible to improve the convenience of a medical worker.

Note that the above-described effects are not necessarily limited, and any of the effects described in the present specification, or other effects that can be grasped from the present specification may be obtained together with the above-described effects or instead of the above-described effects.

### Brief Description of Drawings

FIG. 1 is an explanatory diagram illustrating an example of a configuration of a medical observation system according to an embodiment.
FIG. 2 is a functional block diagram illustrating an example of a configuration of an illumination apparatus according to the embodiment.
FIG. 3 is an explanatory diagram illustrating an example of a configuration of a medical observation apparatus according to the embodiment.
FIG. 4 is an explanatory diagram illustrating an example of a configuration of an imaging device unit included in the medical observation apparatus according to the embodiment.
FIG. 5 is a functional block diagram illustrating an example of a configuration of the medical observation apparatus according to the embodiment.
FIG. 6 is a flowchart illustrating an example of a process of a control method according to the embodiment.
FIG. 7 is a flowchart illustrating another example of the process of the control method according to the embodiment.

### Best Mode for Carrying Out the Invention

Hereinafter, preferred embodiments of the present disclosure will be described in detail with reference to the accompanying drawings. In this specification and the drawings, components having substantially the same functional configuration are denoted by the same reference numerals, and redundant description is omitted.

Hereinafter, description will be given in the following order.
1. Medical Observation System According to Embodiment and Control Method According to Embodiment
   [1] Configuration of Medical Observation System
   [2] Control Method According to Embodiment
   [3] Example of Effect Achieved by Using Control Method According to Embodiment
2. Program According to Embodiment

### (Medical Observation System According to Embodiment and Control Method According to Embodiment)

Hereinafter, a control method according to the embodiment will be described while describing an example of a medical observation system according to the embodiment.

Hereinafter, a case where the medical observation apparatus according to the embodiment performs a process of the control method according to the embodiment, that is, a case where the medical observation apparatus according to the embodiment functions as a medical control apparatus will be mainly described. In the medical observation system according to the embodiment, a device that functions as the medical control apparatus is not limited to the medical observation apparatus according to the embodiment. For example, in the medical observation system according to the embodiment, another device such as a display device, an illumination apparatus, or a navigation apparatus may perform a process of the control method according to the embodiment, and function as a medical control apparatus. In the medical observation system according to the embodiment, any device such as a medical controller which can perform a process of the control method according to the embodiment can function as a medical control apparatus.

### [1] Configuration of Medical Observation System

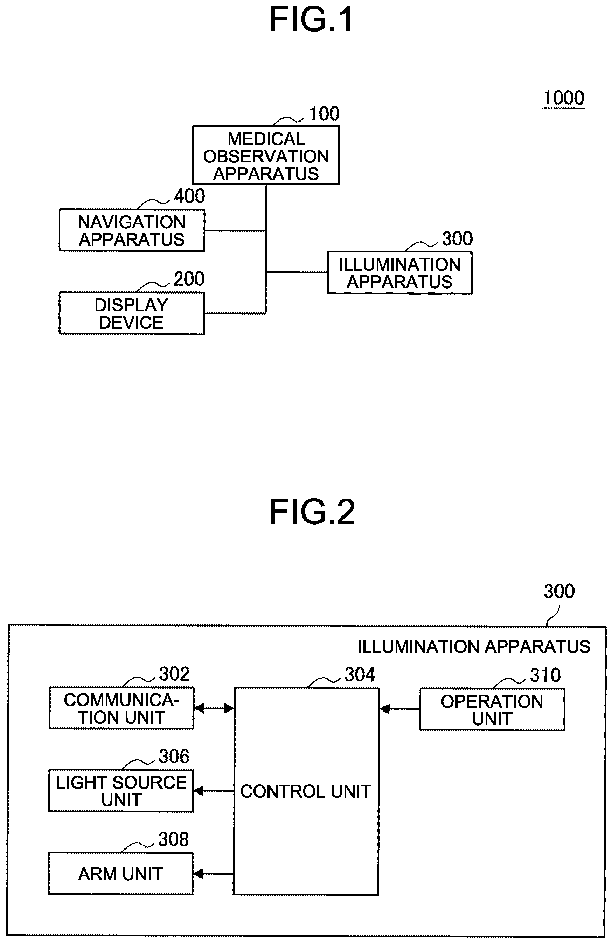

FIG. 1 is an explanatory diagram illustrating an example of a configuration of a medical observation system 1000 according to the embodiment. The medical observation system 1000 includes, for example, a medical observation apparatus 100, a display device 200, an illumination apparatus 300, and a navigation apparatus 400.

The medical observation apparatus 100 is connected to each of the display device 200, the illumination apparatus 300, and the navigation apparatus 400 by any wired communication or any wireless communication.

Note that the medical observation system according to the embodiment is not limited to the example illustrated in FIG. 1.

For example, the medical observation system according to the embodiment may further include a medical control apparatus (not illustrated) that performs a process of the control method according to the embodiment. Further, the medical control apparatus (not illustrated) may have a function of controlling various operations in the medical observation apparatus 100. In the medical observation system 1000 illustrated in FIG. 1, as described below, an example in which the medical observation apparatus 100 has a function of the medical control apparatus (not illustrated) by providing a control unit (described below) that performs a process of the control method according to the embodiment to the medical observation apparatus 100 is illustrated.

As the medical control apparatus (not illustrated), for example, any device that can perform a process of the control method according to the embodiment, such as a "medical controller" or a "computer such as a server", is exemplified. Further, the medical control apparatus (not illustrated) may be, for example, an integrated circuit (IC) that can be incorporated in the above-described device.

Further, the medical observation system according to the embodiment may have a configuration including a plurality of medical observation apparatuses 100 and display devices 200. In a case where there are a plurality of medical observation apparatuses 100, in each of the medical observation apparatuses 100, a process of the control method in the medical observation apparatus 100 described below is performed. Further, in a case where the medical observation system according to the embodiment has a configuration including a plurality of medical observation apparatuses 100 and display devices 200, the medical observation apparatuses 100 may be associated with the display devices 200 on a one-to-one basis, or the plurality of medical observation apparatuses 100 may be associated with one display device 200. In a case where the plurality of medical observation apparatuses 100 are associated with one display device 200, for example, by performing a switching operation or the like, the display device 200 switches the display of a captured image captured by which medical observation apparatus 100, on a display screen. Hereinafter, the captured image captured by the medical observation apparatus 100 is referred to as a "medical captured image".

In addition, FIG. 1 illustrates one illumination apparatus called the illumination apparatus 300, but the number of illumination apparatuses included in the medical observation system according to the embodiment is not limited to one. For example, the medical observation system according to the embodiment may include two or more illumination apparatuses. In a case where there are two or more illumination apparatuses, each of the illumination apparatuses is controlled by a process of the control method in the medical observation apparatus 100 described below.

Further, the medical observation system according to the embodiment may be configured not to include the navigation apparatus 400. Even in a case where the medical observation system according to the embodiment does not include the navigation apparatus 400, by the medical observation apparatus 100 performing a process of the control method according to the embodiment, it is possible to improve the convenience of the medical worker.

Hereinafter, each device constituting the medical observation system 1000 will be described.

### [1-1] display device 200

The display device 200 is display means in the medical observation system 1000, and corresponds to an external display device when viewed from the medical observation apparatus 100. The display device 200 displays various images such as a medical captured image (a moving image or a plurality of still images; the same applies hereinafter) captured by the medical observation apparatus 100 and an image relating to a user interface (UI), on the display screen. Further, the display device 200 may have a configuration capable of performing 3D display by any method. The display in the display device 200 is controlled by, for example, the medical observation apparatus 100 or a medical control apparatus (not illustrated).

In the medical observation system 1000, the display device 200 is installed at any place that can be visually recognized by a person involved in the surgery such as a surgeon in the operating room, such as a wall surface, a ceiling, and a floor surface of the operating room. Examples of the display device 200 include a liquid crystal display, an organic electro-luminescence (EL) display, and a cathode ray tube (CRT) display.

Note that the display device 200 is not limited to the example described above.

For example, the display device 200 may be any wearable device used by a surgeon or the like by wearing the device on his/her body, such as a head-mounted display or an eyewear type device.

The display device 200 is driven by, for example, electric power supplied from an internal power supply such as a battery included in the display device 200 or electric power supplied from a connected external power supply.

### [1-2] Illumination Apparatus 300

The illumination apparatus 300 is an example of illumination in the medical observation system 1000. Examples of the illumination apparatus 300 include any illumination present in the surgery site, other than light sources of an observation device, such as a surgical light or a room light. Examples of the light source of the observation device include a light source of the imaging device included in the medical observation apparatus 100, and a headlight of a surgical loupe, as described below. That is, the illumination apparatus 300 corresponds to an illumination apparatus that irradiates the observation target with illumination light from the outside of the observation device. Further, in the medical observation system 1000, the light source of the observation device and the illumination apparatus 300 are disposed apart from each other.

Here, the illumination apparatus 300 may have any movement mechanism, and may have a configuration in which a spatial position of an illumination device (described below) can be moved. It goes without saying that the illumination apparatus 300 may have a configuration in which the spatial position of the illumination device (described below) is fixed.

FIG. 2 is a functional block diagram illustrating an example of a configuration of the illumination apparatus 300 according to the embodiment. The illumination apparatus 300 includes, for example, a communication unit 302, a control unit 304, a light source unit 306, an arm unit 308, and an operation unit 310. The illumination apparatus 300 is driven by, for example, electric power supplied from an internal power supply such as a battery included in the illumination apparatus 300 or electric power supplied from a connected external power supply.

The communication unit 302 is communication means included in the illumination apparatus 300, and is constituted by a communication device (not illustrated) that plays a role of performing communication with an external device such as the medical observation apparatus 100 in a wireless or wired manner. As the communication device (not illustrated) constituting the communication unit 302, for example, an IEEE 802.15.1 port and a transmission/reception circuit (wireless communication), an IEEE 802.11 port and a transmission/reception circuit (wireless communication), a communication antenna and a radio frequency (RF) circuit (wireless communication) or a local area network (LAN) terminal and a transmission/reception circuit (wired communication). The communication in the communication unit 302 is controlled by the control unit 304, for example.

By providing the communication unit 302, the illumination apparatus 300 can transmit one or both of information indicating the brightness state of the illumination apparatus 300 (for example, data indicating the brightness level of the illumination apparatus 300) and information indicating the posture of the illumination apparatus 300, to the medical observation apparatus 100 (or medical control apparatus (not illustrated)).

The control unit 304 is constituted by one processor or two or more processors (not illustrated) constituted by an arithmetic circuit such as a micro processing unit (MPU) and plays a role of controlling the entire illumination apparatus 300.

Examples of the control in the control unit 304 include control for the communication in the communication unit 302, control for the brightness in the light source unit 306, and control for the movement of the illumination device (not illustrated) in the arm unit 308.

The control unit 304 performs various kinds of control on the basis of a control signal which is transmitted from the medical observation apparatus 100 (an example of the medical control apparatus) and is received by the communication unit 302, for example. Further, the control unit 304 may perform various kinds of control on the basis of an operation on the operation unit 310.

The light source unit 306 is constituted by the illumination device (not illustrated), and functions as light emission means included in the illumination apparatus 300. The illumination device (not illustrated) includes, for example, light sources (not illustrated) such as an incandescent light bulb or a discharge light bulb, a light-emitting diode (LED) light bulb, a light bulb such as a bulb-shaped fluorescent lamp, and a fluorescent lamp. The illumination device (not illustrated) may include a reflector or the like and function as a surgical light.

The arm unit 308 supports the illumination device (not illustrated) constituting the light source unit 306. Further, the arm unit 308 may have a movement mechanism, and may have a configuration in which the spatial position of the illumination device (not illustrated) can be changed by moving the illumination device (not illustrated) constituting the light source unit 306. As the movement mechanism included in the arm unit 308, for example, the same configuration as an arm unit (described below) included in the medical observation apparatus 100 is exemplified. It goes without saying that the example of the movement mechanism included in the arm unit 308 is not limited to the same configuration as the arm unit (described below) included in the medical observation apparatus 100.

The operation unit 310 is operation means included in the illumination apparatus 300, and is constituted by an operation device (not illustrated) that allows an operator such as a medical worker to perform an operation. Examples of the operation device (not illustrated) include a button, a directional key, a rotary selector such as a jog dial, or a combination thereof.

The illumination apparatus 300 has, for example, the configuration illustrated in FIG. 2. Note that the configuration of the illumination apparatus 300 is not limited to the example illustrated in FIG. 2.

For example, the illumination apparatus 300 can be configured not to include the operation unit 310.

### [1-3] Navigation Apparatus 400

The navigation apparatus 400 is medical equipment for realizing a so-called medical navigation system. The navigation apparatus 400 detects, for example, a spatial position of a position detection probe, and causes an image corresponding to the detected spatial position to be displayed on the display screen of the display device 200.

In a case where the medical navigation system is realized by the navigation apparatus 400, a medical worker moves the position detection probe to a position corresponding to a surgical site. The navigation apparatus 400 detects the position of the surgical site of the patient by detecting the spatial position of the position detection probe by any position detection method. Therefore, the medical worker can visually recognize which part of the patient the part being treated corresponds to by viewing the image which is displayed on the display screen of the display device 200 and corresponds to the detected spatial position.

In addition, the medical worker moves the position detection probe to a position where the illumination apparatus 300 is disposed so that it is possible to detect the position of the illumination apparatus 300 in the surgery site.

The navigation apparatus 400 has a function of transmitting positional information indicating the detected spatial position of the position detection probe to an external device such as the medical observation apparatus 100.

The navigation apparatus 400 is driven by, for example, electric power supplied from an internal power supply such as a battery included in the navigation apparatus 400 or electric power supplied from a connected external power supply.

### [1-4] Medical Observation Apparatus 100

The medical observation apparatus 100 is, for example, an electronic imaging medical observation apparatus. For example, in a case where the medical observation apparatus 100 is used at the time of surgery, the surgeon (an example of a user of the medical observation apparatus 100) observes a surgical site (affected part) while referring to the medical captured image which is captured by the medical observation apparatus 100 and is displayed on the display screen of the display device 200, and performs various treatments such as a procedure according to a surgical method on the surgical site.

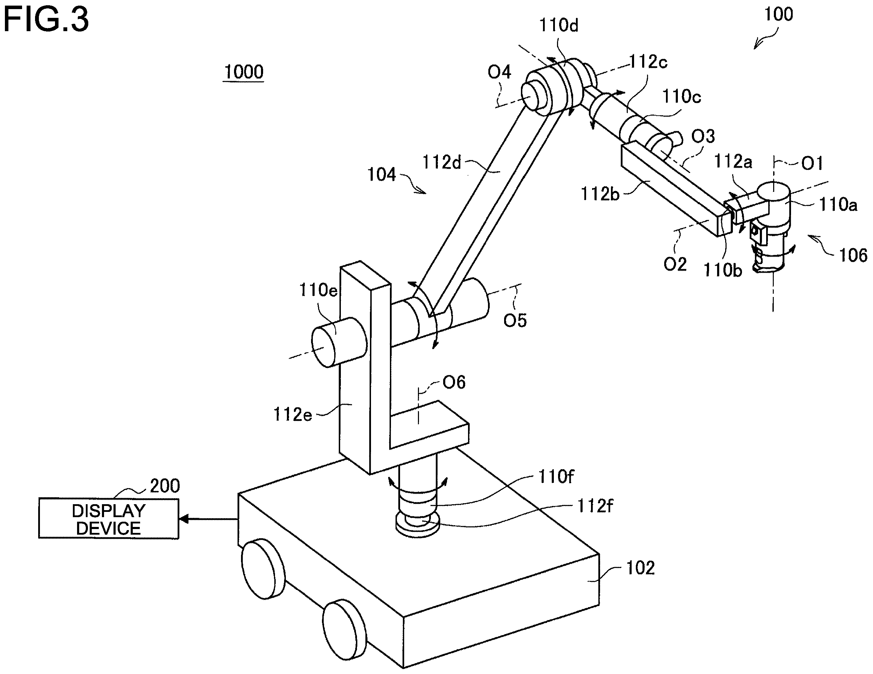

FIG. 3 is an explanatory diagram illustrating an example of the configuration of the medical observation apparatus 100 according to the embodiment. FIG. 3 also illustrates the display device 200.

The medical observation apparatus 100 includes, for example, a base 102, an arm 104, and an imaging device 106.

Although not illustrated in FIG. 3, the medical observation apparatus 100 includes, for example, one processor or two or more processors (not illustrated) each of which is constituted by an arithmetic circuit such as an MPU, a read only memory (ROM; not illustrated), a random access memory (RAM; not illustrated), a recording medium (not illustrated), and a communication device (not illustrated). The medical observation apparatus 100 is driven by, for example, electric power supplied from an internal power supply such as a battery included in the medical observation apparatus 100 or electric power supplied from a connected external power supply.

The processor (not illustrated) functions as a control unit in the medical observation apparatus 100 described below. The ROM (not illustrated) stores control data such as programs and calculation parameters used by the processor (not illustrated). The RAM (not illustrated) temporarily stores a program and the like executed by the processor (not illustrated).

The recording medium (not illustrated) functions as a storage unit (not illustrated) in the medical observation apparatus 100. The recording medium (not illustrated) stores various kinds of data such as various applications and data relating to the control method according to the embodiment, for example. Here, examples of the recording medium (not illustrated) include a magnetic recording medium such as a hard disk, and a nonvolatile memory such as a flash memory. Further, the recording medium (not illustrated) may be detachable from the medical observation apparatus 100.

The communication device (not illustrated) is communication means included in the medical observation apparatus 100, and plays a role of performing communication with an external device such as the display device 200 in a wireless or wired manner. Here, as the communication device (not illustrated), for example, an IEEE 802.15.1 port and a transmission/reception circuit (wireless communication), an IEEE 802.11 port and a transmission/reception circuit (wireless communication), a communication antenna and a RF circuit (wireless communication) or a LAN terminal and a transmission/reception circuit (wired communication).

### [1-4-1] Base 102

The base 102 is a base of the medical observation apparatus 100, and one end of the arm 104 is connected to support the arm 104 and the imaging device 106.

The base 102 is provided with, for example, casters, and the medical observation apparatus 100 is grounded to the floor surface via the casters. By providing the casters, the medical observation apparatus 100 can be easily moved on the floor surface by the casters.

### [1-4-2] Arm 104

The arm 104 is configured such that a plurality of links are connected to each other by joints.

Further, the arm 104 supports the imaging device 106. The imaging device 106 supported by the arm 104 can be moved three-dimensionally, and the position and posture of the moved imaging device 106 are held by the arm 104.

More specifically, the arm 104 is constituted by, for example, a plurality of joints 110a, 110b, 110c, 110d, 110e, and 110f, and a plurality of links 112a, 112b, 112c, 112d, 112e, and 112f that are rotatably connected by the joints 110a, 110b, 110c, 110d, 110e, and 110f. The rotatable range of each of the joints 110a, 110b, 110c, 110d, 110e, and 110f is arbitrarily set in a design stage, a manufacturing stage, or the like such that a desired movement of the arm 104 is realized.

That is, in the medical observation apparatus 100 illustrated in FIG. 3, the six rotation axes (first axis O1, second axis O2, third axis O3, fourth axis O4, fifth axis O5, and sixth axis O6) corresponding to the six joints 110a, 110b, 110c, 110d, 110e, and 110f constituting the arm 104 realize six degrees of freedom regarding the movement of the imaging device 106. More specifically, in the medical observation apparatus 100 illustrated in FIG. 3, the movement with six degrees of freedom of three translational degrees of freedom and three rotational degrees of freedom is realized.

Each of the joints 110a, 110b, 110c, 110d, 110e, and 110f is provided with an actuator (not illustrated), and each of the joints 110a, 110b, 110c, 110d, 110e, and 110f is rotated by the corresponding rotation axis by the driving of the actuator (not illustrated). The driving of the actuator (not illustrated) is controlled by, for example, a processor functioning as a control unit described below, or an external medical control apparatus (not illustrated).

Each of the joints 110a, 110b, 110c, 110d, 110e, and 110f is rotated by the corresponding rotation axis by the driving of the actuator (not illustrated), for example, to realize various operations of the arm 104 such as extending and contracting (folding) of the arm 104.

The joint 110a has a substantially cylindrical shape, and at a distal end portion (lower end portion in FIG. 3) of the joint 110a, the joint supports the imaging device 106 (upper end portion of the imaging device 106 in FIG. 3) such that the imaging device 106 is rotatable around a rotation axis (first axis O1) parallel to the center axis of the imaging device 106. Here, the medical observation apparatus 100 is configured such that the first axis O1 matches the optical axis of the imaging device 106. That is, by rotating the imaging device 106 around the first axis O1 illustrated in FIG. 3, the medical captured image captured by the imaging device 106 becomes an image of which the field of view is changed to rotate.

The link 112a is a substantially rod-shaped member, and fixedly supports the joint 110a. The link 112a extends, for example, in a direction orthogonal to the first axis O1, and is connected to the joint 110b.

The joint 110b has a substantially cylindrical shape, and supports the link 112a such that the link 112a is rotatable around the rotation axis (second axis O2) orthogonal to the first axis O1. The link 112b is fixedly connected to the joint 110b.

The link 112b is a substantially rod-shaped member, and extends in a direction orthogonal to the second axis O2. Further, each of the joint 110b and the joint 110c is connected to the link 112b.

The joint 110c has a substantially cylindrical shape, and supports the link 112b such that the link 112b is rotatable around the rotation axis (third axis O3) orthogonal to each of the first axis O1 and the second axis O2. One end of the link 112c is fixedly connected to the joint 110c.

Here, by rotating the distal end side of the arm 104 (the side on which the imaging device 106 is provided) around the second axis O2 and the third axis O3, the imaging device 106 can be moved such that the position of the imaging device 106 in the horizontal plane is changed. That is, in the medical observation apparatus 100, by the rotation around the second axis O2 and the third axis O3 being controlled, the field of view of the medical captured image can be moved in a plane.

The link 112c is a member having one end having a substantially cylindrical shape and the other end having a substantially rod shape. The joint 110c is fixedly connected to one end side of the link 112c such that the center axis of the joint 110c is the same as the center axis of the substantially cylindrical shape. Further, the joint 110d is connected to the other end side of the link 112c.

The joint 110d has a substantially cylindrical shape, and supports the link 112c such that the link 112c is rotatable around the rotation axis (fourth axis O4) orthogonal to the third axis O3. The link 112d is fixedly connected to the joint 110d.

The link 112d is a substantially rod-shaped member, and extends to be orthogonal to the fourth axis O4. One end of the link 112d is fixedly connected to the joint 110d so as to abut on a side surface of the substantially cylindrical shape of the joint 110d. In addition, the joint 110e is connected to the other end of the link 112d (end opposite to the side to which the joint 110d is connected).

The joint 110e has a substantially cylindrical shape, and supports one end of the link 112d such that the one end of the link 112d is rotatable around the rotation axis (fifth axis O5) parallel to the fourth axis O4. One end of the link 112e is fixedly connected to the joint 110e.

Here, the fourth axis O4 and the fifth axis O5 are rotation axes that can move the imaging device 106 in the vertical direction. By rotating the distal end side of the arm 104 (the side on which the imaging device 106 is provided) around the fourth axis O4 and the fifth axis O5, the position of the imaging device 106 in the vertical direction is changed. Thus, by rotating the distal end side of the arm 104 (the side on which the imaging device 106 is provided) around the fourth axis O4 and the fifth axis O5, the distance between the imaging device 106 and the observation target such as a surgical site of a patient can be changed.

The link 112e is a member configured by the combination of a first member having a substantially L-shape of which one side extends in the vertical direction and the other side extends in the horizontal direction, and a rod-shaped second member extending vertically downward from a portion of the first member extending in the horizontal direction. The joint 110e is fixedly connected to a portion of the first member of the link 112e extending in the vertical direction. The joint 110f is connected to the second member of the link 112e.

The joint 110f has a substantially cylindrical shape, and supports the link 112e such that the link 112e is rotatable around the rotation axis (sixth axis O6) parallel to the vertical direction. The link 112f is fixedly connected to the joint 110f.

The link 112f is a substantially rod-shaped member, and extends in the vertical direction. One end of the link 112f is connected to the joint 110f. The other end of the link 112f (end opposite to the side to which the joint 110f is connected) is fixedly connected to the base 102.

With the arm 104 having the above-described configuration, in the medical observation apparatus 100, six degrees of freedom regarding the movement of the imaging device 106 is realized.

Note that the configuration of the arm 104 is not limited to the above-described example.

For example, each of the joints 110a, 110b, 110c, 110d, 110e, and 110f of the arm 104 may be provided with a brake that restricts the rotation in each of the joints 110a, 110b, 110c, 110d, 110e, and 110f. Examples of the brake according to the embodiment include brakes of any type, such as a mechanically driven brake and an electrically driven electromagnetic brake.

The driving of the brake is controlled by, for example, a processor functioning as a control unit described below, or an external medical control apparatus (not illustrated). By the driving of the brake being controlled, in the medical observation apparatus 100, an operation mode of the arm 104 is set. The operation mode of the arm 104 includes, for example, a fixed mode and a free mode.

Here, the fixed mode according to the embodiment is, for example, an operation mode in which the position and posture of the imaging device 106 are fixed by the rotation of each rotation axis provided to the arm 104 being restricted by the brake. When the arm 104 is in the fixed mode, an operation state of the medical observation apparatus 100 is a fixed state in which the position and posture of the imaging device 106 are fixed.

In addition, the free mode according to the embodiment is an operation mode in which each of the rotation axis provided to the arm 104 is freely rotatable by the brake being released. For example, in the free mode, the position and posture of the imaging device 106 can be adjusted by a direct operation by the surgeon. Here, the direct operation according to the embodiment means, for example, an operation in which the surgeon holds the imaging device 106 with his/her hand and directly moves the imaging device 106.

### [1-4-3] Imaging Device 106

The imaging device 106 is supported by the arm 104, and captures an image of the observation target such as a surgical site of a patient. The imaging device 106 corresponds to an example of an observation device for observing the observation target. The imaging by the imaging device 106 is controlled by, for example, a processor functioning as a control unit described below, or an external control device (not illustrated).

The imaging device 106 has a configuration corresponding to, for example, an electronic imaging microscope.

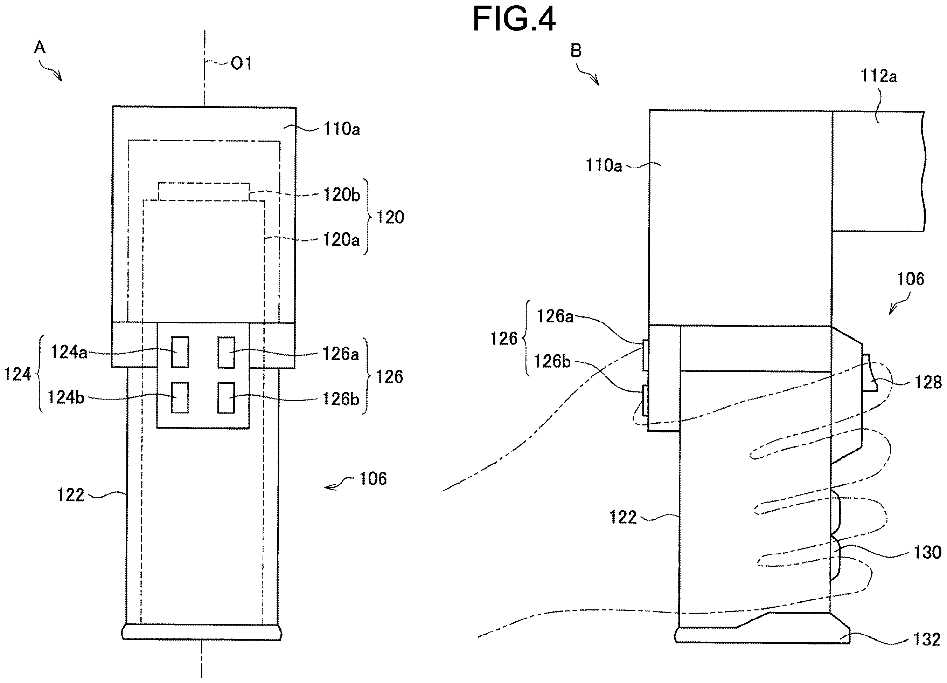

FIG. 4 is an explanatory diagram illustrating an example of a configuration of the imaging device 106 included in the medical observation apparatus 100 according to the embodiment.

The imaging device 106 includes, for example, an imaging member 120 and a cylindrical member 122 having a substantially cylindrical shape, and the imaging member 120 is provided in the cylindrical member 122.

For example, a cover glass (not illustrated) for protecting the imaging member 120 is provided on an opening surface of a lower end (end on the lower side in FIG. 4) of the cylindrical member 122.

Further, for example, a light source (not illustrated) is provided inside the cylindrical member 122, and at the time of imaging, illumination light is emitted from the light source to a subject through the cover glass. The light source (not illustrated) provided in the imaging device 106 is an example of a light source of an observation device, and corresponds to an example of a light source of which the brightness is to be controlled by the process of the control method according to the embodiment. Reflected light (observation light) from the subject irradiated with the illumination light is incident on the imaging member 120 through the cover glass (not illustrated), and thus an image signal indicating the subject (image signal indicating the captured image) is obtained by the imaging member 120.

As the imaging member 120, a configuration used in various known electronic imaging microscope units can be applied.

As an example, the imaging member 120 includes, for example, an optical system 120a and an image sensor 120b including an imaging element that captures an image of the observation target with light passing through the optical system 120a. The optical system 120a is constituted by, for example, optical elements including a mirror and one lens or two or more lenses such as an objective lens, a zoom lens, and a focus lens. Examples of the image sensor 120b include, an image sensor using a plurality of imaging elements such as a complementary metal oxide semiconductor (CMOS) and a charge coupled device (CCD).

The imaging member 120 may have a configuration having a pair of imaging elements, that is, a configuration functioning as a so-called stereo camera. The imaging member 120 has one function or two or more functions generally provided in the electronic imaging microscope unit, such as an auto focus (AF) function, which includes at least a zoom function (one or both of an optical zoom function and an electronic zoom function).

Further, the imaging member 120 may be configured such that imaging with a so-called high resolution such as 4K or 8K is possible. By configuring the imaging member 120 such that imaging with a high resolution is possible, the image can be displayed on the display device 200 having a large display screen of, for example, 50 inches or more while ensuring a predetermined resolution (for example, Full HD image quality), and thus, the visibility of the surgeon viewing the display screen is improved. Further, by configuring the imaging member 120 is such that imaging with a high resolution is possible, a predetermined resolution can be ensured even if the captured image is enlarged by the electronic zoom function to be displayed on the display screen of the display device 200. Further, in a case where a predetermined resolution is ensured by using the electronic zoom function, since it is possible to suppress the performance of the optical zoom function in the imaging device 106, it is possible to simplify the optical system of the imaging device 106, and thus the imaging device 106 can be configured to be smaller.

The imaging device 106 is provided with various operation devices for controlling the operation of the imaging device 106, for example. For example, in FIG. 4, a zoom switch 124, a focus switch 126, and an operation mode change switch 128 are provided to the imaging device 106. It goes without saying that the positions and shapes of the zoom switch 124, the focus switch 126, and the operation mode change switch 128 are not limited to the example illustrated in FIG. 4.

The zoom switch 124 and the focus switch 126 are examples of an operation device for adjusting an imaging condition in the imaging device 106.

The zoom switch 124 includes, for example, a zoom-in switch 124a for increasing the zoom magnification (enlarge magnification) and a zoom-out switch 124b for reducing the zoom magnification. When an operation is performed on the zoom switch 124, the zoom magnification is adjusted so that the zoom is adjusted.

The focus switch 126 includes, for example, a distant view focus switch 126a that increases the focal length to the observation target (subject) and a near view focus switch 126b that decreases the focal length to the observation target. When an operation is performed on the focus switch 126, the focal length is adjusted so that the focus is adjusted.

The operation mode change switch 128 is an example of an operation device for changing the operation mode of the arm 104 in the imaging device 106. When an operation is performed on the operation mode change switch 128, the operation mode of the arm 104 is changed. Examples of the operation mode of the arm 104 include, for example, the fixed mode and the free mode as described above.

Examples of an operation on the operation mode change switch 128 include an operation of pressing the operation mode change switch 128. For example, while the surgeon presses the operation mode change switch 128, the operation mode of the arm 104 is the free mode, and when the surgeon does not press the operation mode change switch 128, the operation mode of the arm 104 is the fixed mode.

The imaging device 106 is provided with, for example, a non-slip member 130 and a protrusion member 132 in order to further improve operability and convenience when an operator, who performs an operation on various operation devices, performs an operation.

The non-slip member 130 is a member provided to prevent an operation body from slipping, for example, when the operator operates the cylindrical member 122 with the operation body such as a hand. The non-slip member 130 is formed of, for example, a material having a large frictional coefficient, and has a structure that is less slippery such as unevenness.

The protrusion member 132 is a member for preventing that the operation body blocks the field of view of the optical system 120a when the operator operates the cylindrical member 122 with the operation body such as a hand, and that the cover glass (not illustrated) is stained due to the operation body touching the cover glass when an operation is performed with the operation body.

It goes without saying that the positions and shapes of the non-slip member 130 and the protrusion member 132 are not limited to the example illustrated in FIG. 4. Further, one or both of the non-slip member 130 and the protrusion member 132 may not be provided to the imaging device 106.

The image signal (image data) generated by the imaging by the imaging device 106 is subjected to an image process in, for example, a processor functioning as a control unit described below. Examples of the image process according to the embodiment include one process or two or more processes among various processes such as gamma correction, white balance adjustment, enlargement or reduction of an image relating to the electronic zoom function, or pixel-to-pixel correction. In a case where the medical observation system according to the embodiment includes a control device (not illustrated) that controls various operations in the medical observation apparatus 100, the image process according to the embodiment may be performed in the control device (not illustrated).

The medical observation apparatus 100 transmits, for example, a display control signal and the image signal on which the above-described image process has been performed, to the display device 200.

By transmitting the display control signal and the image signal to the display device 200, the medical captured image in which the observation target is captured (for example, the captured image in which the surgical site is captured) is displayed on the display screen of the display device 200 by being enlarged or reduced to a desired magnification using one or both of the optical zoom function and the electronic zoom function.

The medical observation apparatus 100 has, for example, the hardware configuration illustrated with reference to FIGS. 3 and 4.

Note that the hardware configuration of the medical observation apparatus according to the embodiment is not limited to the configuration illustrated with reference to FIGS. 3 and 4.

For example, the medical observation apparatus according to the embodiment may not include the base 102, and may have a configuration in which the arm 104 is directly attached to a ceiling or a wall surface of an operating room or the like. For example, in a case where the arm 104 is attached to the ceiling, the medical observation apparatus according to the embodiment has a configuration in which the arm 104 is hung from the ceiling.

FIG. 3 illustrates an example in which the arm 104 is configured such that six degrees of freedom regarding the driving of the imaging device 106 are realized, but the configuration of the arm 104 is not limited to a configuration in which the degree of freedom regarding the driving of the imaging device 106 is six degrees of freedom. For example, it is sufficient that the arm 104 is configured to move the imaging device 106 depending on the application, and the number and disposition of the joints and links, the direction of the drive shaft of the joints, and the like can be appropriately set such that the arm 104 has a desired degree of freedom.

FIGS. 3 and 4 illustrate an example in which various operation devices for controlling the operation of the imaging device 106 are provided to the imaging device 106, but some or all of the operation devices illustrated in FIGS. 3 and 4 may not be provided to the imaging device 106. As an example, various operation devices for controlling the operation of the imaging device 106 may be provided to a portion other than the imaging device 106 constituting the medical observation apparatus according to the embodiment. As another example, various operation devices for controlling the operation of the imaging device 106 may be external operation devices such as a foot switch and a remote controller.

Further, the imaging device 106 may have a configuration in which a plurality of observation modes can be switched. Examples of the observation mode according to the embodiment include, an observation mode in which imaging is performed using natural light, an observation mode in which imaging is performed using special light, and an observation mode in which imaging is performed using an image enhancement observation technique such as narrow band imaging (NBI). The special light according to the embodiment is, for example, light in a specific wavelength band, such as light in a near-infrared wavelength band or light in a fluorescence wavelength band for fluorescence observation using 5-aminolevulinic acid (5-ALA). Hereinafter, the observation mode in which imaging is performed using natural light is referred to as a "first observation mode", and the observation mode in which imaging is performed using special light is referred to as a "second observation mode".

Examples of the configuration of the imaging device 106 in which a plurality of observation modes can be switched include, a "configuration including a filter that transmits light in a specific wavelength band and does not transmit light in other wavelength bands, and a movement mechanism that selectively disposes the filter on an optical path". Examples of the specific wavelength band that the filter according to the embodiment transmits include, a near-infrared wavelength band (for example, a wavelength band of about 0.7 [micrometer] to 2.5 [micrometer]), a fluorescence wavelength band by the fluorescence observation using 5-ALA (for example, a wavelength band of about 0.6 [micrometer] to 0.65 [micrometer]), and a fluorescence wavelength band for indocyanine green (ICG) (for example, a wavelength band of about 0.82 [micrometer] to 0.85 [micrometer]).

Note that the imaging device 106 may be provided with a plurality of filters that transmit different wavelength bands. Further, in the above description, an example in which the filter is disposed on the optical path and imaging is performed with light in a specific wavelength band has been described, but it goes without saying that the configuration of the imaging device 106 for performing imaging with light in a specific wavelength band is not limited to the above-described example.

Note that the medical observation apparatus constituting the medical observation system according to the embodiment is not limited to the electronic imaging medical observation apparatus described with reference to FIGS. 3 and 4. For example, the medical observation apparatus according to the embodiment may be a surgical loupe (binocular loupe). The surgical loupe functioning as the medical observation apparatus according to the embodiment is, for example, "equipment having binocular lenses that are used by being mount on the surgeon's head at the time of surgery and for performing a magnified view of the surgical site and having a headlight between the left and right binocular lenses". The surgical loupe according to the embodiment corresponds to another example of an observation device for observing the observation target. The headlight of the surgical loupe according to the embodiment is another example of the light source of the observation device, and corresponds to another example of the light source of which the brightness is to be controlled by the process of the control method according to the embodiment. Hereinafter, a case where the medical observation apparatus according to the embodiment is the medical observation apparatus 100 illustrated in FIG. 3 will be mainly described.

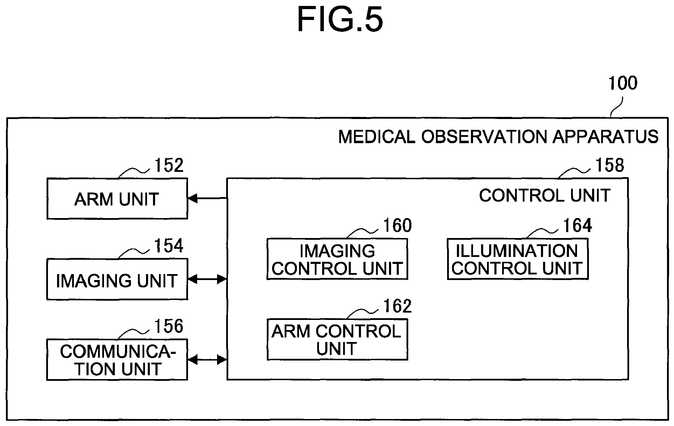

Next, a description will be given using functional blocks of the medical observation apparatus according to the embodiment with the medical observation apparatus 100 illustrated in FIG. 3 as an example. FIG. 5 is a functional block diagram illustrating an example of the configuration of the medical observation apparatus 100 according to the embodiment.

The medical observation apparatus 100 includes, for example, an arm unit 152, an imaging unit 154, a communication unit 156, and a control unit 158.

The arm unit 152 is constituted by the arm 104 and supports the imaging device 106 constituting the imaging unit 154.

The imaging unit 154 is constituted by the imaging device 106, and captures an image of the observation target. The imaging by the imaging unit 154 is controlled by, for example, the control unit 158.

The communication unit 156 is communication means included in the medical observation apparatus 100, and plays a role of performing communication with an external device such as the display device 200, the illumination apparatus 300, and the navigation apparatus 400 in a wireless or wired manner. The communication unit 156 is constituted by, for example, the above-described communication device (not illustrated). The communication in the communication unit 156 is controlled by, for example, the control unit 158.

The control unit 158 is constituted by, for example, the above-described processor (not illustrated), and plays a role of controlling the entire medical observation apparatus 100. Further, the control unit 158 plays a leading role in performing a process of the control method described below. Note that the process of the control method in the control unit 158 may be performed in a distributed manner by a plurality of processing circuits (for example, a plurality of processors).

More specifically, the control unit 158 includes, for example, an imaging control unit 160, an arm control unit 162, and an illumination control unit 164.

The imaging control unit 160 controls the imaging device 106 constituting the imaging unit 154. Examples of the control of the imaging device 106 include, a control of one function or two or more functions generally included in the electronic imaging microscope unit, such as a control of the zoom function (one or both of the optical zoom function and the electronic zoom function), and a control of the AF function.

The arm control unit 162 controls the driving of the arm 104 constituting the arm unit 152. Examples of the control of the driving of the arm 104 include "applying a control signal for controlling the driving to the actuator (not illustrated) corresponding to each of the joints 110a, 110b, 110c, 110d, 110e, and 110f".

The illumination control unit 164 performs a process of the control method described below, and controls illumination in the medical observation system 1000.

The illumination control unit 164 transfers, for example, a control signal to the imaging device 106 constituting the imaging unit 154 to control the brightness of the light source (not illustrated) in the imaging device 106 constituting the imaging unit 154. Note that the brightness of the light source (not illustrated) in the imaging device 106 may be controlled by the imaging control unit 160 constituting the control unit 158. That is, the imaging control unit 160 can also function as a part of the illumination control unit 164.

Further, the illumination control unit 164 transfers, for example, a control signal to the communication device (not illustrated) constituting the communication unit 156, and transmits the control signal to the illumination apparatus 300 to control the brightness of the illumination apparatus 300. The illumination control unit 164 can also control the movement of the position of the illumination apparatus 300 by transmitting, for example, a control signal to the illumination apparatus 300. Note that the communication in the communication unit 156 may be controlled by a communication control unit (not illustrated) constituting the control unit 158.

The control unit 158 plays a leading role in performing the process of the control method according to the embodiment by being provided with, for example, the illumination control unit 164. In addition, the control unit 158 plays a role of controlling the entire medical observation apparatus 100 by being provided with, for example, the imaging control unit 160, the arm control unit 162, and the illumination control unit 164.

Note that the configuration of the control unit 158 is not limited to the example illustrated in FIG. 5.

For example, the control unit 158 can have any configuration according to the method of dividing the functions of the medical observation apparatus 100, such as a configuration according to the method of dividing the process of the control method according to the embodiment.

The medical observation apparatus 100 performs the process of the control method according to the embodiment, which will be described below, with, for example, the configuration illustrated in FIG. 5.

Note that the functional configuration of the medical observation apparatus according to the embodiment is not limited to the configuration illustrated in FIG. 5.

For example, the medical observation apparatus according to the embodiment can include some or all of the imaging control unit 160, the arm control unit 162, and the illumination control unit 164 illustrated in FIG. 5 separately from the control unit 158 (for example, realized by other processing circuits).

Further, the configuration for realizing the process of the control method according to the embodiment in the medical observation apparatus according to the embodiment is not limited to the configuration illustrated in FIG. 5, and for example, the medical observation apparatus according to the embodiment can have a configuration according to the method of dividing the process of the control method according to the embodiment.

Further, for example, in case of performing communication with an external device via an external communication device having the same function and configuration as the communication unit 156, the medical observation apparatus according to the embodiment may not include the communication unit 156.

Further, in a case where the medical observation system according to the embodiment has a configuration including the medical control apparatus (not illustrated) and the medical observation apparatus according to the embodiment is controlled by the medical control apparatus (not illustrated), the medical observation apparatus according to the embodiment may not include the control unit 158.

Here, by providing a control unit having the same function and configuration as the control unit 158, the medical control apparatus (not illustrated) performs a process of the control method according to the embodiment described below, and controls the operation in each component such as the arm unit 152 and the imaging unit 154 included in the medical observation apparatus according to the embodiment. The medical control apparatus (not illustrated) performs communication with the medical observation apparatus according to the embodiment via a communication device provided therein or an external communication device connected thereto, to control the operation in each component included in the medical observation apparatus according to the embodiment.

Further, in a case where the medical observation system according to the embodiment has a configuration including the medical control apparatus (not illustrated) and the medical observation apparatus according to the embodiment is controlled by the medical control apparatus (not illustrated), the medical observation apparatus according to the embodiment can have a configuration not having some of the functions of the control unit 158.

Furthermore, in a case where the medical observation apparatus according to the embodiment is a surgical loupe, the medical observation apparatus according to the embodiment may not include the arm unit 152, the imaging unit 154, and the communication unit 156. In a case where the medical observation apparatus according to the embodiment is a surgical loupe, the control unit 158 constituting the medical observation apparatus according to the embodiment may not include the imaging control unit 160 and the arm control unit 162. In a case where the medical observation apparatus according to the embodiment is a surgical loupe, in the medical observation apparatus according to the embodiment, for example, the illumination control unit 164 controls the brightness of the headlight of the surgical loupe.

### [2] Control Method According to Embodiment

Next, the control method according to the embodiment will be described. Hereinafter, a case where the process of the control method according to the embodiment is performed by the medical observation apparatus 100 (more specifically, for example, the control unit 158 constituting the medical observation apparatus 100) is described as an example. As described above, in the medical observation system according to the embodiment, the process of the control method according to the embodiment may be performed by the medical control apparatus (not illustrated).

### [2-1] Outline of Control Method According to Embodiment

As exemplified in the medical observation system 1000 illustrated in FIG. 1, at the surgery site, there are various illuminations, for example, a light source of the imaging device included in the medical observation apparatus and an illumination apparatus such as a surgical light or a room light. In addition, at the surgery site, various light sources are manually controlled by a medical worker.

However, at the existing surgery site, the following situation has occurred.
- The illuminations present in the surgery site are not linked, and it is not always possible to obtain an optimal medical captured image using the medical observation apparatus. As an example, since the illuminations present in the surgery site are not linked, the surgical site may be difficult to see due to the influence of the movement of medical workers such as a surgeon, an assistant, and a nurse, and the influence of the movement of surgical tools.
- Since the illuminations present in the surgery site are not linked, in order to obtain an optimal medical captured image, for example, it is necessary to individually control the light source of the imaging device included in the medical observation apparatus and the illumination apparatus such as a surgical light. Therefore, the convenience of the medical worker such as a doctor and a nurse may be impaired. As an example, immediately after the start of the surgery, it is common to perform a treatment with the surgical light turned on without using the medical observation apparatus, and it is common to turn off the surgical light at the time of using the medical observation apparatus, but it is necessary for the medical worker to operate the surgical light, which is complicated. As another example, at the time of special light observation, it is necessary to turn off all of the illuminations other than the light source of the imaging device included in the medical observation apparatus, and in a case where normal observation and special light are repeated multiple times, the medical worker has to turn on or off the illuminations every time, which is complicated.
- Since the illuminations present in the surgery site are not linked, the white balance may be adjusted in a state where the surgical light is turned on improperly during the normal setup, and thus a treatment may be performed with a white balance that is not optimal during the surgery.

Therefore, the medical observation apparatus 100 controls the illumination to be controlled present in the medical site such as the surgery site, by the process of the control method according to the embodiment.

Examples of the Illumination to be controlled according to the embodiment include, a light source of the imaging device that captures an image of the observation target, and an illumination apparatus external to the medical observation apparatus 100 such as a surgical light and a room light. Examples of the light source of the imaging device that captures an image of the observation target include a light source of the imaging device 106 included in the medical observation apparatus 100. In a case where the process of the control method according to the embodiment is performed by the medical control apparatus (not illustrated), the light source of the imaging device that captures an image of the observation target may be a light source of the imaging device external to the medical control apparatus (not illustrated).

The medical observation apparatus 100 controls the illuminations to be controlled so as to realize the link of the illuminations to be controlled. In addition, since the medical observation apparatus 100 controls the illuminations to be controlled, it is possible to prevent a situation occurring at the existing surgery site as described above from occurring.

Accordingly, by the medical observation apparatus 100 performing a process of the control method according to the embodiment, it is possible to improve the convenience of the medical worker.

### [2-2] Process of Control Method According to Embodiment

Next, the process of the control method according to the embodiment will be described more specifically. The medical observation apparatus 100 performs, for example, any of the process in following (1) to the process in following (4) as the process of the control method according to the embodiment.

### (1) First Example of Process of Control Method According to Embodiment

The medical observation apparatus 100 performs a control of controlling the brightness of the light source of an observation device for observing the observation target, such as the imaging device 106 and the surgical loupe, and a control of controlling the brightness of the illumination apparatus 300 that irradiates the observation target with illumination light from the outside of the observation device, such as a surgical light, on the basis of the detection result of the brightness relating to imaging. Hereinafter, the control of the brightness of the light source of the observation device may be referred to as "first control", and the control of the brightness of the illumination apparatus 300 may be referred to as "second control". In the following, a case where the observation device is the imaging device that captures an image of the observation target will be mainly described.

Examples of the detection result of the brightness relating to imaging include a detection result of the through-the-lens (TTL) metering which is a metering method using a TTL exposure meter. The TTL metering is a method of measuring light passing through the optical system, and corresponds to a method of measuring light incident on the imaging element such as a CMOS or a CCD.

The TTL exposure meter is provided at a position corresponding to a method of realizing the TTL metering such as viewfinder optical path metering and direct metering, in the imaging device. The medical observation apparatus 100 uses, for example, data indicating the detection result of the TTL exposure meter as a detection result of the brightness relating to imaging. Examples of the data indicating the detection result of the TTL exposure meter include data in which the detection result of the photometry is represented by a unit value representing any brightness such as a light value.

In a case where the detection result of the TTL metering is used as the detection result of the brightness relating to imaging, it is also possible to obtain the detection result of the brightness relating to imaging for each of divided regions obtained by dividing a region of the entire field of view.

For example, the medical observation apparatus 100 compares the detection result of the brightness relating to imaging with a predetermined reference value to determine whether the brightness relating to imaging is too bright or too dark. Then, the medical observation apparatus 100 performs the first control and the second control according to the determination result of the brightness.

More specifically, the medical observation apparatus 100 performs the first control with priority over the second control. Performing the first control with priority over the second control means that, for example, the first control is performed first when the brightness relating to imaging is adjusted, and the second control is performed in a case where the first control cannot be performed. The case where the first control cannot be performed refers to a case where the brightness of the light source of the imaging device cannot be adjusted by the first control.

The reason that the medical observation apparatus 100 performs the first control with priority over the second control is that, for example, controlling the brightness of the light source of the imaging device has a greater effect of adjusting the brightness relating to imaging than controlling the brightness of the illumination apparatus 300.

It goes without saying that the medical observation apparatus 100 can perform the second control with priority over the first control, and the medical observation apparatus 100 can perform the first control and the second control simultaneously.

### (2) Second Example of Process of Control Method According to Embodiment

The medical observation apparatus 100 controls the movement of the illumination apparatus 300 such as a surgical light in addition to the process according to the first example illustrated in (1) described above. Hereinafter, the control of the movement of the illumination apparatus 300 may be referred to as "third control".

For example, the medical observation apparatus 100 performs the third control in a case where the first control and the second control cannot be performed. The case where the second control cannot be performed refers to a case where the brightness of the illumination apparatus 300 cannot be adjusted by the second control. That is, the medical observation apparatus 100 performs the third control in a case where the process according to the first example illustrated in (1) described above cannot be performed.

In a case where the first control and the second control cannot be performed, the medical observation apparatus 100 controls the movement of the illumination apparatus 300 such as a surgical light on the basis of the positional information acquired from the navigation apparatus 400, for example.

Since the light source of the imaging device 106 included in the medical observation apparatus 100 is not a coaxial illumination, in a case where a doctor performs a small incision and treats a deep part, the light intensity may be insufficient. Here, it is possible to make the medical captured image look bright by the image process such as increasing the gain, but the resolution may be significantly reduced.

In a case where the doctor performs a small incision and treats a deep part as described above, it is possible to compensate for the above-described insufficiency of the light intensity by using the illumination apparatus such as a surgical light, as an auxiliary apparatus. However, in a case where the illumination apparatus is used as an auxiliary apparatus, light does not pass deep into the hole of the opening unless the illumination apparatus is aligned with the axis of the hole of the opening. In addition, it is complicated to manually align the illumination apparatus with the axis of the hole of the opening.

Therefore, the medical observation apparatus 100 moves the illumination apparatus 300 such as a surgical light on the basis of the positional information.

As described above, the position indicated by the positional information acquired from the navigation apparatus 400 can indicate the position of the surgical site in the patient and the position of the illumination apparatus 300, respectively. Further, in a case where the surgery is performed on the patient, the imaging device 106 included in the medical observation apparatus 100 captures an image of the surgical site in the patient.

Therefore, when the medical observation apparatus 100 moves the illumination apparatus 300 on the basis of the positional information, align the illumination apparatus 300 can be automatically aligned with the axis of the hole of the opening. In addition, when the medical observation apparatus 100 moves the illumination apparatus 300 on the basis of the positional information, an effect as the coaxial illumination is achieved. Note that the medical observation apparatus 100 can further control the movement of the illumination apparatus 300 on the basis of information indicating the posture of the illumination apparatus 300.

Note that the process according to the second example in which the third control is performed is not limited to the example described above.

For example, in a case where a shadow on the surgical site is detected from the captured image, the medical observation apparatus 100 may perform the third control. After the start of moving the illumination apparatus 300, the medical observation apparatus 100 stops the movement of the illumination apparatus 300 when the shadow is no longer detected from the captured image.

Examples of the captured image according to the third control include a medical captured image captured by the imaging device that captures an image of the observation target, such as the imaging device 106, or a captured image captured by a surgery site imaging device provided at the surgery site. The surgery site imaging device is, for example, an imaging device that captures an image of the entire surgery site (or a specific region of the surgery site), and may be referred to as an overhead camera.

In a case where the captured image according to the third control is a captured image captured by the surgery site imaging device, the medical observation apparatus 100 performs an arbitrary object detection process on the captured image to detect an object such as people or medical equipment, and may move the illumination apparatus 300 on the basis of the detection result of the object. As described above, by moving the illumination apparatus 300 on the basis of the detection result of the object, more efficient movement of the illumination apparatus 300 is realized.

Note that the medical observation apparatus 100 can perform the third control using both the medical captured image captured by the imaging device that captures an image of the observation target, such as the imaging device 106, and the captured image captured by the surgery site imaging device provided at the surgery site.

### (3) Third Example of Process of Control Method According to Embodiment

The medical observation apparatus 100 performs the second control to correspond to the observation mode of the medical observation apparatus 100 including the imaging device that captures an image of the observation target. That is, the medical observation apparatus 100 controls the brightness of the illumination apparatus 300 in conjunction with the observation mode of the medical observation apparatus 100.

As an example, the medical observation apparatus 100 turns off the illumination apparatus 300 when the observation mode is switched from the first observation mode in which imaging is performed using natural light to the second observation mode in which imaging is performed using special light. As another example, the illumination apparatus 300 is turned on when the observation mode is switched from the second observation mode in which imaging is performed using special light to the first observation mode in which imaging is performed using natural light.

For example, as described above, by automatically switching the state of the illumination apparatus 300 in conjunction with the switching of the observation mode in the medical observation apparatus 100, it is not necessary for the medical worker to perform the on/off operation of the illumination apparatus 300 every time the observation mode is switched. Therefore, by performing the process according to the third example, it is possible to improve the convenience of the medical worker.

It goes without saying that in a case where the medical observation apparatus 100 has a function of performing the process according to the third example, the state of the illumination apparatus 300 can be manually switched by turning off the function.

### (4) Fourth Example of Process of Control Method According to Embodiment

The medical observation apparatus 100 can perform "a process obtained by combining the process according to the first example illustrated in (1) described above or the process according to the second example illustrated in (2) described above, and the process according to the third example illustrated in (3) described above".

The medical observation apparatus 100 performs, for example, any of the process in (1) described above to the process in (4) described above as the process of the control method according to the embodiment.

Note that the process of the control method according to the embodiment is not limited to the example described above.

For example, the medical observation apparatus 100 may perform a predetermined notification on the basis of a combination of the state of the light source of the imaging device that captures an image of the observation target, such as the imaging device 106, and the state of the illumination apparatus 300. The medical observation apparatus 100 performs a predetermined notification in addition to the process in (1) described above to the process in (4) described above, or independently of the process in (1) described above to the process in (4) described above.

For example, the medical observation apparatus 100 notifies of the notification content by a "visual notification by displaying a character or image (for example, an icon) corresponding to the notification content on the display screen of the display device 200", an "audible notification by outputting audio (including music) corresponding to the notification content from an audio output device such as a speaker", or a combination thereof. The audio output device may be included in the medical observation apparatus 100 or may be a device external to the medical observation apparatus 100.

As an example of the predetermined notification, the medical observation apparatus 100 notifies of the notification content indicating re-acquisition of the white balance when the illumination condition is changed from the illumination condition at the time of acquisition of the white balance. Data indicating the illumination condition at the time of acquisition of the white balance is stored in, for example, a storage unit (not illustrated).

As another example of the predetermined notification, the medical observation apparatus 100 notifies of the notification content indicating that the illumination condition is an illumination condition which is not normally used, in a case where the illumination condition does not match any illumination condition of one illumination condition or two or more illumination conditions stored in the storage unit (not illustrated). As an example, the medical observation apparatus 100 performs a notification "in a case where the light source of the imaging device 106 included in the medical observation apparatus 100 is off and the surgical light is on".

Note that the predetermined notification according to the embodiment is not limited to the example described above.

For example, the medical observation apparatus 100 may further perform a notification to select whether to change the illumination state, in addition to the above-described predetermined notification.

In a case where an operation for changing the illumination state is detected after the notification for selection is performed, the medical observation apparatus 100 automatically adjusts settings for each of the light source of the imaging device that captures an image of the observation target, such as the imaging device 106, and the illumination apparatus 300. The medical observation apparatus 100 performs the above-described automatic adjustment on the basis of an operation on the operation device included in the medical observation apparatus 100 or the operation device external to the medical observation apparatus 100 such as a remote controller.

### [2-3] Example of Process of Control Method According to Embodiment

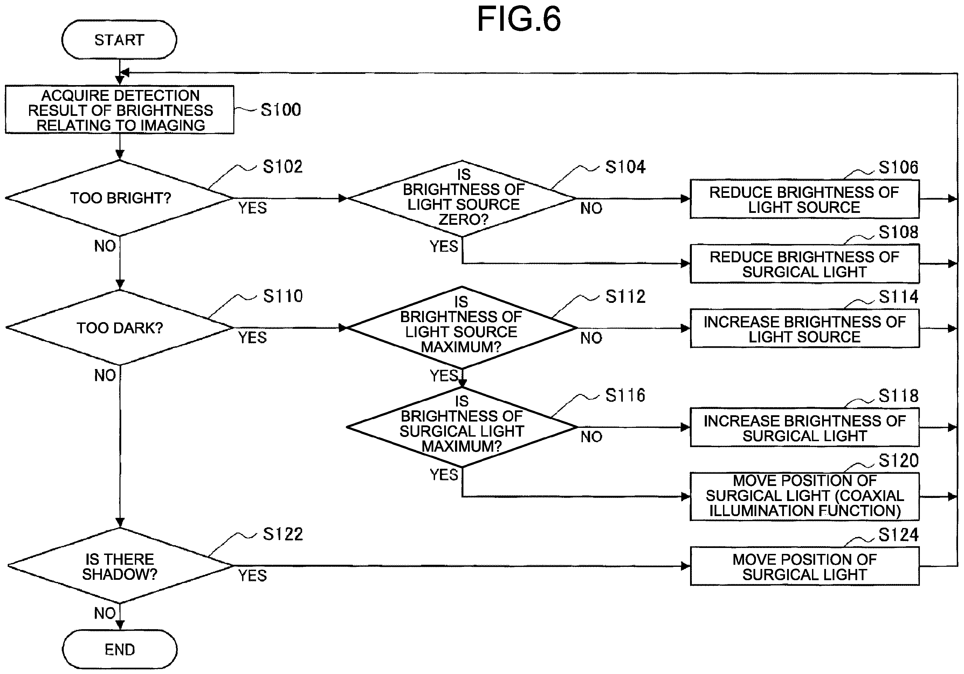

FIG. 6 is a flowchart illustrating an example of the process of the control method according to the embodiment. The process illustrated in FIG. 6 corresponds to an example of the process according to the fourth example illustrated in (4) described above. FIG. 6 illustrates an example in which the medical observation apparatus 100 controls the light source of the imaging device 106 included in the medical observation apparatus 100 and the surgical light (an example of the illumination apparatus 300). Hereinafter, the light source of the imaging device 106 may be simply referred to as a "light source".

The medical observation apparatus 100 acquires a detection result of the brightness relating to imaging (S100). The medical observation apparatus 100 acquires the data indicating the detection result of the TTL exposure meter from the imaging device 106 to acquire the detection result of the brightness relating to imaging.

The medical observation apparatus 100 determines whether the brightness is too high on the basis of the detection result of the brightness relating to imaging (S102). The medical observation apparatus 100 compares the value indicated by the detection result of the brightness relating to imaging (hereinafter, referred to as a "detection value") with a set first threshold to determine whether the brightness is too high. The medical observation apparatus 100 determines that the brightness is too high, for example, in a case where the detection value is larger than the first threshold (or in a case where the detection value is equal to or larger than the first threshold). The first threshold may be a fixed threshold that is set in advance, or may be a variable value that can be changed by an operation of the medical worker or the like.

In a case where it is determined in step S102 that the brightness is too high, the medical observation apparatus 100 determines whether the brightness of the light source is zero (S104).

In a case where it is determined in step S104 that the brightness of the light source is not zero, the medical observation apparatus 100 reduces the brightness of the light source (S106). The medical observation apparatus 100 reduces the brightness of the light source by lowering the brightness of the light source by one level. It goes without saying that the medical observation apparatus 100 can lower the brightness of the light source by a plurality of levels.

In a case where it is determined in step S104 that the brightness of the light source is zero, that is, in a case where the first control cannot be performed, the medical observation apparatus 100 reduces the brightness of the surgical light (S108). The medical observation apparatus 100 reduces the brightness of the surgical light by lowering the brightness of the surgical light by one level. It goes without saying that the medical observation apparatus 100 can lower the brightness of the surgical light by a plurality of levels.

When the process in step S106 or the process in step S108 is performed, the medical observation apparatus 100 repeats the process from step S100.

In a case where it is determined in step S102 that the brightness is not too high, the medical observation apparatus 100 determines whether the brightness is too dark on the basis of the detection result of the brightness relating to imaging (S110). The medical observation apparatus 100 compares the detection value indicated by the detection result of the brightness relating to imaging with a set second threshold (second threshold < first threshold) to determine whether the brightness is too dark. For example, in a case where the detection value is smaller than the second threshold (or in a case where the detection value is equal to or smaller than the second threshold), the medical observation apparatus 100 determines that the brightness is too dark. The second threshold may be a fixed threshold that is set in advance, or may be a variable value that can be changed by an operation of the medical worker or the like.

In a case where it is determined in step S110 that the brightness is too dark, the medical observation apparatus 100 determines whether the brightness of the light source is maximum (S112).

In a case where it is determined in step S112 that the brightness of the light source is not maximum, the medical observation apparatus 100 increases the brightness of the light source (S114). The medical observation apparatus 100 increases the brightness of the light source by increasing the brightness of the light source by one level. It goes without saying that the medical observation apparatus 100 can increase the brightness of the light source by a plurality of levels.

In a case where it is determined in step S112 that the brightness of the light source is maximum, that is, in a case where the first control cannot be performed, the medical observation apparatus 100 determines whether the brightness of the surgical light is maximum (S116).

In a case where it is determined in step S116 that the brightness of the surgical light is not maximum, the medical observation apparatus 100 increases the brightness of the surgical light (S118). The medical observation apparatus 100 increases the brightness of the surgical light by increasing the brightness of the surgical light by one level. It goes without saying that the medical observation apparatus 100 can increase the brightness of the surgical light by a plurality of levels.

Further, in a case where it is determined in step S116 that the brightness of the surgical light is maximum, that is, in a case where the first control and the second control cannot be performed, the medical observation apparatus 100 performs the third control to move the position of the surgical light (S120). The movement of the position of the surgical light in step S120 corresponds to movement for achieving an effect as the coaxial illumination.

When the process in step S114, the process in step S118, or the process in step S120 is performed, the medical observation apparatus 100 repeats the process from step S100.

In a case where it is determined in step S102 that the brightness is not too dark, the medical observation apparatus 100 determines whether there is a shadow (S122). The medical observation apparatus 100 determines that there is a shadow in a case where a shadow on the surgical site is detected from the captured image. As described above, examples of the captured image relating to the determination in step S122 include a medical captured image captured by the imaging device 106, and a captured image captured by the surgery site imaging device provided at the surgery site.

In a case where it is determined in step S122 that there is a shadow, the medical observation apparatus 100 performs the third control to move the position of the surgical light (S124). Then, the medical observation apparatus 100 repeats the process from step S100.

In a case where it is determined in step S122 that there is no shadow, the medical observation apparatus 100 ends the process in FIG. 6. Note that the process illustrated in FIG. 6 can be repeatedly performed.

The medical observation apparatus 100 performs, for example, the process illustrated in FIG. 6 as the process of the control method according to the embodiment.

Note that an example of the process of the control method according to the embodiment is not limited to the example illustrated in FIG. 6.

For example, it is also possible for the medical observation apparatus 100 to detect a feature of an image such as a luminance distribution and a shape from the medical captured image and to control the light source of the imaging device 106 that captures an image of the observation target and the illumination apparatus 300 such that a partial area including the detected feature portion is optimized.

In addition, the medical observation apparatus 100 can perform various processes such as not performing the processes of step S116 and S120 and not performing the processes of step S122 and step S124 in FIG. 6, for example.

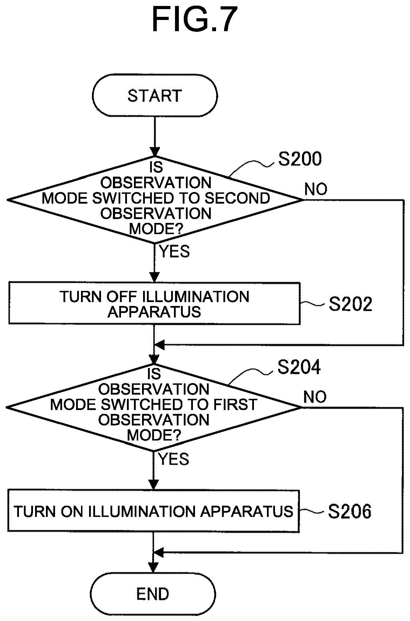

FIG. 7 is a flowchart illustrating another example of the process of the control method according to the embodiment. The process illustrated in FIG. 7 corresponds to an example of the process according to the third example illustrated in (3) described above. The process illustrated in FIG. 7 is repeatedly performed, for example, regularly or irregularly. Hereinafter, the process illustrated in FIG. 7 will be described using a case in which the medical observation apparatus 100 controls the illumination apparatus 300 as an example. In FIG. 7, the illumination apparatus 300 is simply referred to as an "illumination apparatus".

The medical observation apparatus 100 determines whether the observation mode is switched from the first observation mode in which imaging is performed using natural light to the second observation mode in which imaging is performed using special light (S200). The medical observation apparatus 100 determines whether the observation mode is switched from the first observation mode to the second observation mode on the basis of, for example, "a setting state of the observation mode" and "a signal corresponding to the operation, which is transmitted from the operation device for switching the observation mode". The medical observation apparatus 100 specifies the setting state of the observation mode by referring to, for example, "data indicating the setting state of the observation mode (for example, a flag indicating the setting state of the observation mode)" stored in the recording medium (not illustrated). The operation device for switching the observation mode may be an operation device included in the medical observation apparatus 100 or an external operation device. It goes without saying that the method of determining the observation mode according to the embodiment is not limited to the example described above.

In a case where it not determined in step S200 that the observation mode is not switched from the first observation mode to the second observation mode, the medical observation apparatus 100 performs the process from step S204 described below.

In a case where it is determined in step S200 that the observation mode is switched from the first observation mode to the second observation mode, the medical observation apparatus 100 turns off the illumination apparatus 300 (S204). In a case where the method of determining the observation mode according to the embodiment is a method using data indicating the setting state of the observation mode as described above, the medical observation apparatus 100 updates the data indicating the setting state of the observation mode to data indicating the second observation mode.

In a case where it is determined in step S200 that the observation mode is not switched from the first observation mode to the second observation mode or in a case where the process of step S204 is performed, the medical observation apparatus 100 determines whether the observation mode is switched from the second observation mode to the first observation mode (S204). Similar to step S200, the medical observation apparatus 100 determines whether the observation mode is switched from the second observation mode to the first observation mode on the basis of, for example, "a setting state of the observation mode" and "a signal corresponding to the operation, which is transmitted from the operation device for switching the observation mode".

In a case where it is determined in step S204 that the observation mode is not switched from the second observation mode to the first observation mode, the medical observation apparatus 100 ends the process illustrated in FIG. 7.

In a case where it is determined in step S204 that the observation mode is switched from the second observation mode to the first observation mode, the medical observation apparatus 100 turns on the illumination apparatus 300 (S206). In a case where the method of determining the observation mode according to the embodiment is a method using data indicating the setting state of the observation mode as described above, the medical observation apparatus 100 updates the data indicating the setting state of the observation mode to data indicating the first observation mode. Then, the medical observation apparatus 100 ends the process illustrated in FIG. 7.

### [3] Example of Effect Achieved by Using Control Method According to Embodiment

By using the control method according to the embodiment, for example, the following effects can be achieved. It goes without saying that the effects achieved by using the control method according to the embodiment are not limited to the examples described below.
- The medical observation apparatus 100 functioning as a medical control apparatus grasps the state of illuminations present in a medical site such as an operating room, and performs centralized control. Therefore, since the medical observation apparatus 100 can dynamically control the illumination condition of the light source of the imaging device 106 included in the medical observation apparatus 100 and the illumination condition of the illumination apparatus 300, it is possible to always provide an illumination environment for the medical worker to easily perform observation.
- The medical observation apparatus 100 functioning as a medical control apparatus can automatically switch, depending on the observation method, illuminations present in the medical site, such as the light source of the imaging device 106 included in the medical observation apparatus 100 and the illumination apparatus 300 such as a surgical light or a room light. Therefore, since the switching which has been manually operated is automated, it is possible to reduce the burden on the surgeon and the circulating nurse and reduce the operation time, and further to reduce the burden on the patient who receives the medical treatment.
- The medical observation apparatus 100 functioning as a medical control apparatus can monitor a normal white balance, and perform a notification in a case where the white balance deviates from the normal white balance. Therefore, it is expected to reduce the surgeon's misjudgment due to a slight difference in color.
- In a case where an optical medical observation apparatus is used, since the dimming performance of eyes is extremely high, it is not necessary for the optical medical observation apparatus to have a dimming function (actually, which is used with the maximum light intensity). In contrast, the medical observation apparatus 100 which is an electronic imaging medical observation apparatus is not used by looking into the eyepiece as in the case where an optical medical observation apparatus is used. Since the medical observation apparatus 100 functioning as a medical control apparatus has a function of performing the first control of controlling the brightness of the light source of the imaging device 106 that performs imaging, it is possible to provide an illumination environment for the medical worker to easily perform observation without depending on the dimming performance of eyes.

### (Program According to Embodiment)

A program for causing a computer system to function as the medical observation apparatus according to the embodiment (or the control device according to the embodiment) (for example, a program capable of executing the process of the control method according to the embodiment) is executed by a processor or the like in the computer system, and thereby it is possible to improve the convenience of the medical worker. Here, examples of the computer system according to the embodiment include a single computer or a plurality of computers. A series of processes of the control method according to the embodiment is performed by the computer system according to the embodiment.

Further, the program for causing the computer system to function as the medical observation apparatus according to the embodiment (or the control device according to the embodiment) is executed by a processor or the like in the computer system, and thereby it is possible to achieve effects achieved by the display realized by the above-described process of the control method according to the embodiment.

The preferred embodiments of the present disclosure have been described above in detail with reference to the accompanying drawings, but the technical scope of the present disclosure is not limited to such examples. It is apparent that a person having ordinary knowledge in the technical field of the present disclosure can conceive of various kinds of changes or modifications within the scope of the technical idea described in the claims, and it is naturally understood that the changes or modifications also belong to the technical scope of the present disclosure.

For example, in the above description, a case where a program (computer program) for causing the computer system to function as the medical observation apparatus according to the embodiment is provided is described, but the embodiment can further provide also a recording medium in which the program is stored.

The configuration described above illustrates an example of the embodiment, and naturally belongs to the technical scope of the present disclosure.

Further, the effects described in the present specification are merely illustrative or exemplary and are not limited. That is, the technology according to the present disclosure can achieve other effects that are apparent to those skilled in the art from the description of the present specification in addition to the above-described effects or instead of the above-described effects.

Note that the following configurations also belong to the technical scope of the present disclosure.
(1) A medical control apparatus including an illumination control unit that performs a first control of controlling brightness of a light source of an imaging device that captures an image of an observation target and a second control of controlling brightness of an illumination apparatus, based on a detection result of brightness relating to imaging.
(2) The medical control apparatus according to (1), in which the illumination control unit performs the first control with priority over the second control.
(3) The medical control apparatus according to (2), in which the illumination control unit performs the second control in a case where the first control is not able to be performed.
(4) The medical control apparatus according to any one of (1) to (3), in which the illumination control unit further performs a third control of controlling movement of the illumination apparatus.
(5) The medical control apparatus according to (4), in which the illumination control unit performs the third control in a case where the first control and the second control are not able to be performed.
(6) The medical control apparatus according to (4) or (5), in which the illumination control unit controls the movement of the illumination apparatus based on positional information acquired from a navigation apparatus.
(7) The medical control apparatus according to any one of (4) to (6), in which the illumination control unit performs the third control in a case where a shadow is detected from a captured image.
(8) The medical control apparatus according to (7), in which the captured image is a medical captured image which is captured by the imaging device, or a captured image which is captured by a surgery site imaging device provided in a surgery site.
(9) The medical control apparatus according to any one of (1) to (8), in which the illumination control unit performs the second control to correspond to an observation mode of a medical observation apparatus including the imaging device.
(10) The medical control apparatus according to (9), in which the illumination control unit is configured to turn off the illumination apparatus when the observation mode is switched from a first observation mode in which imaging is performed using natural light to a second observation mode in which imaging is performed using special light, and turn on the illumination apparatus when the observation mode is switched from the second observation mode to the first observation mode.
(11) The medical control apparatus according to any one of (1) to (10), in which the illumination control unit performs a predetermined notification based on a combination of a state of the light source of the imaging device and a state of the illumination apparatus.
(12) The medical control apparatus according to any one of (1) to (11), further including an arm configured by connecting a plurality of links by joints; and the imaging device supported by the arm.
(13) The medical control apparatus according to any one of (1) to (12), in which the imaging device and the illumination apparatus are disposed apart from each other.
(14) The medical control apparatus according to any one of (1) to (13), in which the illumination apparatus is a surgical light provided in a surgery site.
(15) A control method executed by a medical control apparatus, the control method including a step of performing a first control of controlling brightness of a light source of an imaging device that captures an image of an observation target and a second control of controlling brightness of an illumination apparatus, based on a detection result of brightness relating to imaging.

Further, the following configurations also belong to the technical scope of the present disclosure.
(1) A medical control apparatus including an illumination control unit that performs, based on a detection result of brightness relating to imaging, a first control of controlling brightness of a light source of an observation device for observing an observation target and a second control of controlling brightness of an illumination apparatus that irradiates the observation target with illumination light from an outside of the observation device.
(2) The medical control apparatus according to (1), wherein the observation device is an imaging device that captures an image of the observation target, and
   the illumination control unit controls brightness of a light source of the imaging device.
(3) The medical control apparatus according to (2), wherein the illumination control unit performs the first control with priority over the second control.
(4) The medical control apparatus according to (3), wherein the illumination control unit performs the second control in a case where the first control is not able to be performed.
(5) The medical control apparatus according to any one of (2) to (4), wherein the illumination control unit further performs a third control of controlling movement of the illumination apparatus.
(6) The medical control apparatus according to (5), wherein the illumination control unit performs the third control in a case where the first control and the second control are not able to be performed.
(7) The medical control apparatus according to (5) or (6), wherein the illumination control unit controls the movement of the illumination apparatus based on positional information acquired from a navigation apparatus.
(8) The medical control apparatus according to any one of (5) to (7), wherein the illumination control unit performs the third control in a case where a shadow is detected from a captured image.
(9) The medical control apparatus according to (8), wherein the captured image is a medical captured image which is captured by the imaging device, or a captured image which is captured by a surgery site imaging device provided in a surgery site.
(10) The medical control apparatus according to any one of (2) to (9), wherein the illumination control unit performs the second control to correspond to an observation mode of a medical observation apparatus including the imaging device.
(11) The medical control apparatus according to (10), wherein the illumination control unit:
   turns off the illumination apparatus when the observation mode is switched from a first observation mode in which imaging is performed using natural light to a second observation mode in which imaging is performed using special light; and
   turns on the illumination apparatus when the observation mode is switched from the second observation mode to the first observation mode.
(12) The medical control apparatus according to any one of (2) to (11), wherein the illumination control unit performs a predetermined notification based on a combination of a state of the light source of the imaging device and a state of the illumination apparatus.
(13) The medical control apparatus according to any one of (2) to (12), further including:
   an arm configured by connecting a plurality of links by joints; and
   the imaging device supported by the arm.
(14) The medical control apparatus according to any one of (2) to (13), wherein the imaging device and the illumination apparatus are disposed apart from each other.
(15) The medical control apparatus according to any one of (2) to (14), wherein the illumination apparatus is a surgical light provided in a surgery site.
(16) A control method executed by a medical control apparatus, the control method including a step of performing, based on a detection result of brightness relating to imaging, a first control of controlling brightness of a light source of an observation device for observing an observation target and a second control of controlling brightness of an illumination apparatus that irradiates the observation target with illumination light from an outside of the observation device.

### Reference Signs List

100 MEDICAL OBSERVATION APPARATUS
102 BASE
104 ARM
106 IMAGING DEVICE
110a, 110b, 110c, 110d, 110e, 110f JOINT
112a, 112b, 112c, 112d, 112e, 112f LINK
120 IMAGING MEMBER
122 CYLINDRICAL MEMBER
124 ZOOM SWITCH
126 FOCUS SWITCH
128 OPERATION MODE CHANGE SWITCH
152, 308 ARM UNIT
154 IMAGING UNIT
156, 302 COMMUNICATION UNIT
158, 304 CONTROL UNIT
160 IMAGING CONTROL UNIT
162 ARM CONTROL UNIT
164 ILLUMINATION CONTROL UNIT
200 DISPLAY DEVICE
300 ILLUMINATION APPARATUS
306 LIGHT SOURCE UNIT
310 OPERATION UNIT
400 NAVIGATION APPARATUS
1000 MEDICAL OBSERVATION SYSTEM

## Claims

1. A medical control apparatus comprising an illumination control unit that performs, based on a detection result of brightness relating to imaging, a first control of controlling brightness of a light source of an observation device for observing an observation target and a second control of controlling brightness of an illumination apparatus that irradiates the observation target with illumination light from an outside of the observation device.

2. The medical control apparatus according to claim 1, wherein the observation device is an imaging device that captures an image of the observation target, and
the illumination control unit controls brightness of a light source of the imaging device.

3. The medical control apparatus according to claim 2, wherein the illumination control unit performs the first control with priority over the second control.

4. The medical control apparatus according to claim 3, wherein the illumination control unit performs the second control in a case where the first control is not able to be performed.

5. The medical control apparatus according to claim 2, wherein the illumination control unit further performs a third control of controlling movement of the illumination apparatus.

6. The medical control apparatus according to claim 5, wherein the illumination control unit performs the third control in a case where the first control and the second control are not able to be performed.

7. The medical control apparatus according to claim 5, wherein the illumination control unit controls the movement of the illumination apparatus based on positional information acquired from a navigation apparatus.

8. The medical control apparatus according to claim 5, wherein the illumination control unit performs the third control in a case where a shadow is detected from a captured image.

9. The medical control apparatus according to claim 8, wherein the captured image is a medical captured image which is captured by the imaging device, or a captured image which is captured by a surgery site imaging device provided in a surgery site.

10. The medical control apparatus according to claim 2, wherein the illumination control unit performs the second control to correspond to an observation mode of a medical observation apparatus including the imaging device.

11. The medical control apparatus according to claim 10, wherein the illumination control unit:
turns off the illumination apparatus when the observation mode is switched from a first observation mode in which imaging is performed using natural light to a second observation mode in which imaging is performed using special light; and
turns on the illumination apparatus when the observation mode is switched from the second observation mode to the first observation mode.

12. The medical control apparatus according to claim 2, wherein the illumination control unit performs a predetermined notification based on a combination of a state of the light source of the imaging device and a state of the illumination apparatus.

13. The medical control apparatus according to claim 2, further comprising:
an arm configured by connecting a plurality of links by joints; and
the imaging device supported by the arm.

14. The medical control apparatus according to claim 2, wherein the imaging device and the illumination apparatus are disposed apart from each other.

15. The medical control apparatus according to claim 2, wherein the illumination apparatus is a surgical light provided in a surgery site.

16. A control method executed by a medical control apparatus, the control method comprising a step of performing, based on a detection result of brightness relating to imaging, a first control of controlling brightness of a light source of an observation device for observing an observation target and a second control of controlling brightness of an illumination apparatus that irradiates the observation target with illumination light from an outside of the observation device.
